# EUROPÄISCHE PATENTSCHRIFT

(11) **EP 2 796 863 B1**
(45) Veröffentlichungstag und Bekanntmachung des Hinweises auf die Patenterteilung: **06.07.2016**
(21) Anmeldenummer: 14154275.3
(22) Anmeldetag: 07.02.2014
(51) Int. Cl.: G01N 23/083, G01N 33/44

(54) **Verfahren zur Identifizierung eines Wechsels von Gummimischungen bei der Herstellung von Reifenbauteilen mit einem Extruder**
Method for identifying a change of rubber mixtures in the production of tyre components with an extruder
Procédé d'identification d'un changement de mélanges de caoutchouc lors de la fabrication de composants de pneumatiques à l'aide d'une extrudeuse

(30) Priorität: 25.04.2013 DE 102013104182
(43) Veröffentlichungstag der Anmeldung: 29.10.2014
(73) Patentinhaber: Continental Reifen Deutschland GmbH, 30165 Hannover (DE)
(72) Erfinder: Lacayo-Pineda, Jorge, 31535 Neustadt (DE); du Bois, Andre, 30657 Hannover (DE); Pavlinek, Vladimir, 76502 Otrokovice (CZ); Cermak, Roman, 76001 Zlin (CZ)
(74) Vertreter: Widjaja, Wira

(56) Entgegenhaltungen:
- GB-A- 2 047 887

## Beschreibung

Die Erfindung betrifft ein Verfahren zur Identifizierung eines Wechsels von Gummimischungen mit einem Extruder zur Herstellung von Reifenbauteilen.

Es ist bekannt, z.B. Laufstreifen für Fahrzeugreifen mit Extrudern herzustellen. Beim kontinuierlichen Wechsel von Gummimischungen während des Extrusionsprozesses fällt im Allgemeinen ein bestimmter Anteil von Gummimaterial an, der als Ausschuss aus dem Herstellungsprozess aussortiert werden muss. Der Grund dafür liegt darin, dass beim Austausch der alten Gummimischung durch eine neue Gummimischung ein kontinuierlicher Wechsel im Extruder erfolgt. Dadurch kommt aus dem Extruder für eine bestimmte Zeitspanne ein Materialgemisch aus alter und neuer Gummimischung. Ein Problem beim kontinuierlichen Wechsel der Gummimischungen besteht darin, die entsprechenden Materialübergänge genau zu identifizieren, um dadurch den Materialausschuss möglichst gering zu halten. Die GB 2 047 887 A offenbart ein bekanntes Verfahren für eine Materialuntersuchung.

Der Erfindung liegt die Aufgabe zugrunde, ein Verfahren zu schaffen, mit dem eine Identifizierung der Materialübergänge bei einem Mischungswechsel am Extruder erfolgen kann.

Gelöst wird die Aufgabe gemäß Anspruchs 1 mit einem Verfahren mit folgenden Schritten:
a) Bereitstellen einer ersten Gummimischung mit unterschiedlichen Mischungsbestandteilen mit einem Extruder,
b) Kontinuierliches Auswechseln der ersten Gummimischung durch eine zweite Gummimischung am Extruder,
   wobei über eine bestimmte Zeitspanne aus der Extruderöffnung ein Gummimaterial aus der ersten und zweiten Gummimischung gefördert wird,
c) Positionierung einer Röntgenvorrichtung mit einer Signalquelle zur Erzeugung einer Röntgenstrahlung und einem Detektor zum Empfangen einer Röntgenstrahlung im Ausgangsbereich des Extruders,
d) Bestrahlung der Oberfläche des aus dem Extruder austretenden Gummimaterials mit der Röntgenstrahlung,
   wobei zumindestens ein Teil der Strahlung durch das Gummimaterial absorbiert wird,
e) Empfangen der durch das Gummimaterial durchgelassen Strahlung mit dem Detektor,
f) Auswertung der empfangenen Röntgenstrahlung und Ermittlung eines Absorptionskoeffizienten,
g) Bestimmen der Änderung der gemessenen Absorptionskoeffizienten während des Wechsels von der ersten zur zweiten Gummimischung,
   wobei die Absorptionskoeffizienten durch die Mischungsspezifikation des herzustellenden Reifenbauteiles festgelegt sind,
h) Identifizierung von zwei Übergangsbereichen im Gummimaterial im Austritt des Extruders,
   wobei der erste Übergang der Mischungsübergang von der ersten Gummimischung zur Mischung aus erster und zweiter Gummimischung ist und
   der zweite Übergang der Mischungsübergang von der Mischung aus erster und zweiter Gummimischung zur zweiten Mischung ist,
i) Identifizierung der Gummimateriallänge aus erster und zweiter Gummimischung
j) Entfernen des unreinen Gummimaterials aus erster und zweiter Gummimischung aus dem Herstellungsprozeß,
k) kontinuierliche Weiterführung des Extrusionsprozesses mit der zweiten Gummimischung.

Ein Vorteil der Erfindung ist insbesondere darin zu sehen, dass durch das Verfahren der Materialausschuss bei einem Wechsel der Gummimischungen am Extruder auf ein Minimum reduziert wird. Mit dem erfindungsgemäßen Messverfahren lassen sich die beiden Übergangsbereiche im Gummimaterial am Austritt des Extruders mit einer hohen Genauigkeit identifizieren. Da das Messverfahren ebenfalls kontaktlos erfolgt, wird die herkömmliche Prozessgeschwindigkeit in keiner Weise beeinträchtigt. Jede reale Gummimischung hat einen spezifischen Absorptionskoeffizienten, der durch den Betrag der jeweiligen Absorption bestimmt wird. Über diesen Absorptionskoeffizienten lassen sich Gummimischungen voneinander unterscheiden. Mit dem Röntgenmessverfahren gemäß Anspruch 1 lassen sich die Absorptionskoeffizienten mit einer hohen Genauigkeit bestimmen. Über die identifizierten Materialübergänge lässt sich anschließend die Materiallänge bestimmten, die als Materialausschuss aus dem Extruderprozess entfernt werden muss.

In einer vorteilhaften Ausführung Ausführung der Erfindung ist vorgesehen, dass die Röntgenvorrichtung mit einer automatisierten Datenerfassung gekoppelt ist, wobei die bei Schritt i) ermittelte Gummimateriallänge aus erster und zweiter Gummimischung in einer Datenbank abgespeichert wird. Auf diese Weise ist für jede neue Gummipaarung immer nur eine Messung erforderlich. Bei einem Wechsel der Gummimischungen auf eine bereits erfassten Paarung kann auf die Daten der Datenbank zugegriffen werden.

In einer weiteren vorteilhaften Ausführung der Erfindung ist vorgesehen, dass zumindest bei Schritt c) die Röntgenvorrichtung eine mobile Einheit ist und nur bei einem in der Datenbank noch nicht erfassten Wechsel der Gummimischung vor der Austrittsöffnung des Extruders positioniert wird.

In einer weiteren vorteilhaften Ausführung der Erfindung ist vorgesehen, dass bei Schritt c) die Röntgenvorrichtung eine stationäre Einheit ist, die vor der Austrittsöffnung des Extruders positioniert wird. Bei diesem Ausführungsbeispiel würde die Röntgenvorrichtung stationär vor dem Extruder angeordnet. Dadurch kann der Messvorgang jederzeit durchgeführt werden.

In einer weiteren vorteilhaften Ausführung der Erfindung ist vorgesehen, dass bei Schritt h) bei beiden Übergangsbereichen ein vorgegebener Toleranzbereich berücksichtigt wird, wobei der Toleranzbereich einen geringen Mischungsanteil vorgibt, mit der die erste oder zweite Gummimischung jeweils verunreinigt sein darf. Auf diese Weise lässt sich der Materialausschuss beim Herstellungsprozess mit dem Extruder reduzieren.

In einer weiteren vorteilhaften Ausführung der Erfindung ist vorgesehen, dass bei Schritt j) das Entfernen des unreinen Gummimaterials aus erster und zweiter Gummimischung im Wesentlichen automatisiert erfolgt. Dadurch lässt sich eine hohe Prozessgeschwindigkeit erreichen.

Anhand eines Ausführungsbeispiels soll die Erfindung näher erläutert werden. Es zeigt:

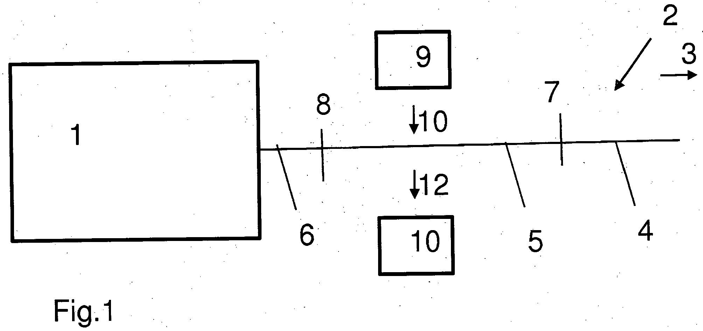

Fig. 1: ein Ausführungsbeispiel für das Verfahren,

Mit dem schematisch dargestellten Extruder 1 wird beispielsweise Laufstreifenmaterial für Fahrzeugreifen hergestellt.

Der Extruder 1 könnte jedoch auch ein Extruder für ein beliebiges Gummibauteil sein, z.B. ein Extruder zur Herstellung von Gummischläuchen etc.

Das Laufstreifenmaterial wird in Förderrichtung 3 aus einer Austrittsöffnung am Extruder 1 herausgeführt. Bei einem Wechsel der Gummimischungen für das Laufstreifenmaterial wird kontinuierlich eine neue zweite Gummimischung dem Extruder 1 zugeführt. Da sich in dem Extruder 1 noch die alte erste Gummimischung befindet, kommt es über eine bestimmte Zeitspanne zu einer Vermischung von erster alter Gummimischung und neuer zweiter Gummimischung. Das Gummimaterial 4 besteht aus der ersten Gummimischung und wird in Förderrichtung 3 bewegt. Der erste Mischungsübergang 7 ist schematisch dargestellt. Dieser Mischungsübergang 7 stellt den Übergang von der ersten Gummimischung zur Mischung aus erster und zweiter Gummimischung dar. Anschließend steigt die Konzentration der zweiten Gummimischung kontinuierlich an bis zum zweiten Mischungsübergang 8. Der zweite Mischungsübergang 8 stellt den Übergang der Mischung aus erster und zweiter Gummimischung sowie zweiter Gummimischung dar. Das aus dem Extruder austretende Gummimaterial 6 besteht nach einer bestimmten Zeitspanne im Wesentlichen aus der zweiten neuen Gummimischung. Das Gummimaterial 5 aus ersten und zweiter Gummimischung stellt Materialausschuss dar, welches aus dem Herstellungsprozess entfernt werden muss. Mit dem Messverfahren gemäß Anspruch 1 werden die Mischungsübergänge 7 und 8 mit einer hohen Genauigkeit identifiziert. Die Röntgenvorrichtung für das Messverfahren ist im Bereich vor der Austrittsöffnung des Extruders 1 positioniert. Es kann sich dabei um ein mobiles System handeln, welches nur bei der Messung einer neuen Gummipaarung eingesetzt wird. Die Signalquelle 9 der Röntgenvorrichtung emittiert eine Röntgenstrahlung 11, die auf die Materialoberfläche des Gummimaterials 2 bzw. der entsprechenden Gummimischung auftrifft. Unterhalb der Signalquelle 9 ist ein Detektor 10 zum Empfangen der Röntgenstrahlung angeordnet. Die Röntgenstrahlung 11 wird zum Teil durch das Gummimaterial absorbiert. Die nicht absorbierte Röntgenstrahlung 12 geht durch das Gummimaterial hindurch und wird von dem Detektor 10 detektiert. Jede Gummimischung besitzt einen spezifischen Absorptionskoeffizienten. Durch die Änderung der Absorptionskoeffizienten lassen sich die beiden Mischungsübergänge 7 und 8 identifizieren. Das Gummimaterial zwischen den beiden Mischungsübergängen 7 und 8 stellt Materialausschuss dar und wird aus dem Herstellungsprozess entfernt.

### Bezugszeichenliste

### (ist Teil der Beschreibung)

- 1: Extruder, z.B. zur Herstellung von Laufstreifen
- 2: Aus dem Extruder austretende Gummimischung
- 3: Förderrichtung
- 4: Gummimaterial aus erster Gummimischung
- 5: Gummimaterial aus erster und zweiter Gummimischung
- 6: Gummimaterial aus im Wesentlichen zweiter Gummimischung
- 7: Erster Mischungsübergang von der ersten Gummimischung zur Mischung aus erster und zweiter Gummimischung
- 8: Zweiter Mischungsübergang von der Mischung aus erster und zweiter Gummimischung sowie zweiter Mischung
- 9: Signalquelle der Röngtenvorrichtung
- 10: Detektor zum Empfangen der Röngtenstrahlung
- 11: Röngtenstrahlung
- 12: Durchgelassene Röntgenstrahlung

## Patentansprüche

1. Verfahren zur Identifizierung eines Wechsels von Gummimischungen mit einem Extruder (1) zur Herstellung von Reifenbauteilen mit folgenden Schritten :
a) Bereitstellen einer ersten Gummimischung mit unterschiedlichen Mischungsbestandteilen mit einem Extruder (1),
b) Kontinuierliches Auswechseln der ersten Gummimischung durch eine zweite Gummimischung am Extruder,
wobei über eine bestimmte Zeitspanne aus der Extruderöffnung ein Gummimaterial (5) als Gemisch aus der ersten und zweiten Gummimischung gefördert wird,
c) Positionierung einer Röntgenvorrichtung mit einer Signälquelle (9) zur Erzeugung einer Röntgenstrahlung (11) und einem Detektor (10) zum Empfangen einer Röntgenstrahlung (12) im Ausgangsbereich des Extruders (1),
d) Bestrahlung der Oberfläche des aus dem Extruder (1) austretenden Gummimaterials (2, 4, 5, 6) mit der Signalquelle (9) der Röntgenvorrichtung,
wobei zumindestens ein Teil der Bestrahlung (11) durch das Gummimaterial (2, 4, 5, 6) adsorbiert wird,
e) Empfangen der durch das Gummimaterial (2, 4, 5, 6) durchgelassen Strahlung (12) mit dem Detektor (10) zum Empfangen der Röngtenstrahlung,
f) Auswertung der empfangenen Röntgenstrahlung (12) und Ermittlung eines Absorptionskoeffizienten,
g) Bestimmen der Änderung der gemessenen Absorptionskoeffizienten während des Wechsels von der ersten zur zweiten Gummimischung,
wobei die Absorptionskoeffizienten durch die Mischungsspezifikation des herzustellenden Gummibauteiles festgelegt sind,
h) Identifizierung von zwei Übergangsbereichen (7, 8) im Gummimaterial (2) im Austritt des Extruders (1),
wobei der erste Übergang der Mischungsübergang (7) von der ersten Gummimischung zur Mischung aus erster und zweiter Gummimischung ist und
der zweite Übergang der Mischungsübergang (8) von der Mischung aus erster und zweiter Gummimischung zur zweiten Gummmischung ist,
i) Identifizierung der Gummimaterials aus erster und zweiter Gummimischung,
j) Entfernen des unreinen Gummimaterials (5) aus erster und zweiter Gummimischung aus dem Herstellungsprozeß,
k) kontinuierliche Weiterführung des Extrusionsprozesses mit der zweiten Gummimmischung.

2. Verfahren nach Anspruch 1,
**dadurch gekennzeichnet, dass**
die Röntgenvorrichtung mit einer automatisierten Datenerfassung gekoppelt ist, wobei die bei den Schritten h) und i) ermittelten Daten in einer Datenbank abgespeichert werden.

3. Verfahren nach einem der vorhergehenden Ansprüche,
**dadurch gekennzeichnet, dass**
zumindestens bei Schritt c) die Röntgenvorrichtung eine mobile Einheit ist und nur bei einem bei einem noch nicht erfassten Wechsel vor der Austrittsöffnung des Extruders (1) positioniert wird.

4. Verfahren nach einem der vorhergehenden Ansprüche,
**dadurch gekennzeichnet, dass**
bei Schritt c) die Röntgenvorrichtung eine stationäre Einheit ist, die vor der Austrittsöffnung des Extruders (1) positioniert wird.,

5. Verfahren nach einem der vorhergehenden Ansprüche,
**dadurch gekennzeichnet, dass**
bei Schritt h) bei beiden Übergangsbereichen (7, 8) ein vorgegebener Toleranzbereich berücksichtigt wird,
wobei der Toleranzbereich einen geringen Mischungsanteil vorgibt, mit der die erste oder zweite Gummimischung jeweils verunreinigt sein darf.

6. Verfahren nach einem der vorhergehenden Ansprüche,
**dadurch gekennzeichnet, dass**
bei Schritt j) das Entfernen des unreinen Gummimaterials (5) aus erster und zweiter Gummimischung im Wesentlichen automatisiert erfolgt.

## Claims

1. Method for identifying a change of rubber mixtures with an extruder (1) for producing tyre components, comprising the following steps:
a) providing a first rubber mixture, comprising different mixture constituents, with an extruder (1),
b) continuously changing the first rubber mixture for a second rubber mixture at the extruder, wherein a rubber material (5) is delivered from the extruder opening over a certain time period as a mixture comprising the first and second rubber mixtures,
c) positioning an x-ray device comprising a signal source (9) for generating an x-radiation (11) and a detector (10) for receiving an x-radiation (12) in the outlet region of the extruder (1),
d) irradiating the surface of the rubber material (2, 4, 5, 6) emerging from the extruder (1) with the signal source (9) of the x-ray device,
wherein at least part of the irradiation (11) is adsorbed by the rubber material (2, 4, 5, 6),
e) receiving the radiation (12) allowed through the rubber material (2, 4, 5, 6) with the detector (10) for receiving the x-radiation,
f) evaluating the received x-radiation (12) and determining an absorption coefficient,
g) determining the change in the measured absorption coefficients during the change from the first rubber mixture to the second rubber mixture, wherein the absorption coefficients are established by the specification of the mixture of the rubber compound to be produced,
h) identifying two transitional regions (7, 8) in the rubber material (2) in the outlet of the extruder (1),
wherein the first transition is the mixture transition (7) from the first rubber mixture to the mixture comprising the first and second rubber mixtures and
the second transition is the mixture transition (8) from the mixture comprising the first and second rubber mixtures to the second rubber mixture,
i) identifying the rubber material comprising the first and second rubber mixtures,
j) removing the unpure rubber material (5) from the first and second rubber mixtures from the production process,
k) continuously carrying on with the extrusion process with the second rubber mixture.

2. Method according to Claim 1,
**characterized in that**
the x-ray device is coupled to an automated data acquisition, wherein the data determined in steps h) and i) are stored in a database.

3. Method according to one of the preceding claims,
**characterized in that**
at least in step c), the x-ray device is a mobile unit and is only positioned in front of the outlet opening of the extruder (1) when there is a not yet recorded change.

4. Method according to one of the preceding claims,
**characterized in that**
in step c), the x-ray device is a stationary unit, which is positioned in front of the outlet opening of the extruder (1).

5. Method according to one of the preceding claims,
**characterized in that**
in step h), a prescribed tolerance range is taken into account for both transitional regions (7, 8), wherein the tolerance range prescribes a small mixture component with which the first or second rubber mixture may be respectively contaminated.

6. Method according to one of the preceding claims,
**characterized in that**
in step j), the removal of the unpure rubber material (5) from the first and second rubber mixtures takes place in a substantially automated manner.

## Revendications

1. Procédé d'identification d'un changement de mélanges de caoutchouc réalisés avec une extrudeuse (1) de fabrication de composants de bandages de roue, le procédé présentant les étapes suivantes :
a) préparer à l'aide d'une extrudeuse (1) un premier mélange de caoutchouc présentant différents composants de mélange,
b) sur l'extrudeuse, remplacer en continu le premier mélange de caoutchouc par un deuxième mélange de caoutchouc,
un matériau de caoutchouc (5) constitué d'un mélange du premier et du deuxième mélange de caoutchouc étant expulsé de l'orifice de l'extrudeuse pendant une durée définie,
c) placer au niveau de la sortie de l'extrudeuse (1) un ensemble à rayons X présentant une source (9) de signaux qui produit un rayonnement X (11) et un détecteur (10) qui reçoit qui reçoit un rayonnement X (12),
d) irradier la surface du matériau de caoutchouc (2, 4, 5, 6) qui sort de l'extrudeuse (1) à l'aide de la source (9) de signaux de l'ensemble à rayons X,
au moins une partie de l'irradiation (11) étant adsorbée par le matériau de caoutchouc (2, 4, 5, 6),
e) recevoir à l'aide du détecteur (10) qui reçoit qui reçoit le rayonnement X le rayonnement (12) qui a traversé le matériau de caoutchouc (2, 4, 5, 6),
f) évaluer le rayonnement X (12) qui a été reçu et déterminer un coefficient d'absorption,
g) déterminer la modification des coefficients d'absorption qui ont été mesurés pendant le passage du premier au deuxième mélange de caoutchouc,
les coefficients d'absorption étant définis par la spécification de mélange du composant de caoutchouc à fabriquer,
h) identifier deux plages de transition (7, 8) dans le matériau de caoutchouc (2) à la sortie de l'extrudeuse (1),
la première transition étant la transition (7) de mélange entre le premier mélange de caoutchouc et le mélange formé par le premier et le deuxième mélange de caoutchouc et
la deuxième transition étant la transition (8) de mélange entre le mélange formé par le premier et le deuxième mélange de caoutchouc et le deuxième mélange de caoutchouc,
i) identifier le matériau de caoutchouc formé par le premier et le deuxième mélange de caoutchouc,
j) extraire du processus de fabrication le matériau de caoutchouc (5) non pur formé par le premier et le deuxième mélange de caoutchouc et
k) poursuivre l'opération d'extrusion avec le deuxième mélange de caoutchouc.

2. Procédé selon la revendication 1, **caractérisé en ce que** l'ensemble à rayons X est raccordé à une saisie automatique de données, les données déterminées aux étapes h) et i) étant conservées dans une banque de données.

3. Procédé selon l'une des revendications précédentes, **caractérisé en ce qu'**au moins à l'étape c), l'ensemble à rayons X est une unité mobile qui n'est positionnée devant l'ouverture de sortie de l'extrudeuse (1) que pour un changement qui n'a pas encore été saisi.

4. Procédé selon l'une des revendications précédentes, **caractérisé en ce qu'**à l'étape c), l'ensemble à rayons X est une unité fixe qui est positionnée devant l'ouverture de sortie de l'extrudeuse (1).

5. Procédé selon l'une des revendications précédentes, **caractérisé en ce qu'**à l'étape h), une plage prédéterminée de tolérance est prise en compte pour les deux plages de transition (7, 8), la plage de tolérance définissant une petite teneur de mélange qui peut encrasser le premier ou le deuxième mélange de caoutchouc.

6. Procédé selon l'une des revendications précédentes, **caractérisé en ce qu'**à l'étape j), l'extraction du matériau de caoutchouc (5) non pur formé par le premier et le deuxième mélange de caoutchouc s'effectue de manière essentiellement automatique.
